# EUROPÄISCHE PATENTSCHRIFT

(11) **EP 2 902 011 B1**
(45) Veröffentlichungstag und Bekanntmachung des Hinweises auf die Patenterteilung: **17.01.2018**
(21) Anmeldenummer: 14153835.5
(22) Anmeldetag: 04.02.2014
(51) Int. Cl.: A61K 8/60, A61K 8/46, A61Q 5/02, A61Q 9/02, A61Q 19/10, C11D 1/83, C11D 17/00, A61K 8/44, C11D 1/90, A61Q 11/00, C11D 1/94, C11D 1/28

(54) **WÄSSRIGE TENSID-ZUSAMMENSETZUNGEN**
AQUEOUS TENSIDE COMPOSITIONS
COMPOSITIONS DE TENSIOACTIFS AQUEUSES

(43) Veröffentlichungstag der Anmeldung: 05.08.2015
(73) Patentinhaber: BASF SE, 67056 Ludwigshafen am Rhein (DE)
(72) Erfinder: Brunn, Claudia, 40597 Düsseldorf (DE); Behler, Ansgar, 46240 Bottrop (DE); Stanislowski, Detlev, 40822 Mettmann (DE); Barbenheim, Monika, 46240 Bottrop (DE)
(74) Vertreter: BASF IP Association

(56) Entgegenhaltungen:
- EP-A1- 2 277 860
- DE-A1-102007 038 029
- US-A1- 2009 227 482
- US-A1- 2012 208 898
- Anonymous: "Foaming Hand Wash", GNDP (Global New Products Database ), 1. Juni 2006 (2006-06-01), Seiten 1-2, XP055127005, INTERNET Gefunden im Internet: URL:http://www.gnpd.com/sinatra/recordpage /536439/from_search/YiRfThoNgE/with_sort/2 / [gefunden am 2014-07-04]

## Beschreibung

### Gebiet der Erfindung

Die vorliegende Erfindung betrifft wäßrige Tensid-Zusammensetzungen mit einem Gehalt an alpha-Sulfofettsäuredisalzen sowie speziellen Amidoalkylbetainen.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel eine ausreichende Wasserlöslichkeit, eine gute Verträglichkeit mit möglichst vielen der in der Kosmetik eingesetzten Wirk- und Hilfsstoffen, ein gutes Schaumvermögen und eine gute Verdickbarkeit erwünscht. Des Weiteren besteht ein Bedarf an anionischen Tensiden, die zumindest teilweise aus biogenen Quellen und speziell auch nachwachsenden Rohstoffen hergestellt werden können. Weiterhin besteht auch ein Bedarf an Tensiden, die keine alkoxilierten Gruppen aufweisen und die somit insbesondere den Einsatz von Ethylenoxid zu ihrer Herstellung überflüssig machen.

Die sogenannten alpha-Sulfofettsäuredisalze ("Disalze") sind eine bekannte Tensidklasse, die jedoch eine sehr schlechte Wasserlöslichkeit aufweisen (vergl. z.B. F.Schambil und M.J.Schwuger, Tenside Surf. Det. 27 (1990), 6 S. 380-385): So beträgt etwa die Wasserlöslichkeit von C14-Di-Na-Salz bei 20°C lediglich 0,7% (vergl. die Graphik auf S. 381). Dies ist für die Praxis, etwa kosmetische Zubereitungen unbefriedigend gering.

Dass Disalze sich generell als Viskositäts-Senker für unterschiedliche anionische Tensidformulierungen eignen, ist seit langem bekannt, vergleiche z.B. DE-A-1216470, DE-A-1221391, DE-A-1218646 und DE-A-1,225799. In all diesen Fällen handelt es sich um den Einsatz von Disalz als Hydrotrop, d.h. als Substanz mit verflüssigenden, viskositäts-senkenden Eigenschaften. Dementsprechend würde der Fachmann, der auf der Suche nach Viskositäts-steigernden Substanzen ist, auf Grund der Lehre dieser Dokumente die dort offenbarten Disalze nicht in Betracht ziehen.

WO-A-92/15660 offenbart flüssige Reinigungsmittel mit einem Gehalt an sulfoölsauren Disalzen. Es wird offenbart, dass sulfoölsauren Disalze in der Lage sind, die Viskosität von Tensiden oder Tensidgemischen für Reinigungsmittel - insbesondere auf Basis von Fettalkylsulfaten, Fettalkylethersulfaten, Alkylpolyglucosiden und Fettsäuremonoethanolamiden - zu senken und zwar ebenso effektiv oder sogar besser wie durch Zusatz von Ethanol oder Hydrotropen (Seite 2, zweiter Absatz). In diesem Zusammenhang wird darauf hingewiesen, dass sulfoölsaure Disalze sehr gut wasserlöslich sind, ganz im Gegensatz zum alpha-Sulfostearinsäure-Disalz, das schwer wasserlöslich ist (vergleiche den die Seiten 2 und 3 überbrückenden Absatz). Auf Seite 3, Zeilen 3-6, wird schließlich ausgeführt, dass C₁₂₋₁₄-Disalze auf Basis von gesättigten Fettsäuren viskositätssenkend sind. Dementsprechend würde der Fachmann, der auf der Suche nach Viskositäts-steigernden Substanzen ist, auf Grund der Lehre dieses Dokumentes Disalze nicht in Betracht ziehen.

WO-A-2011/049932 beschreibt flüssige Reinigungszusammensetzungen mit einem Gehalt an Disalzen und Betainen. Dabei ist erfindungswesentlich, dass es sich bei den Betainen um Alkylbetaine handelt. Von der Verwendung von Amidoalkylbetainen und insbesondere der Alkylamidopropylbetaine wird ausdrücklich abgeraten. So heißt es auf S. 9, Zeilen 1 bis 4, ausdrücklich, dass Formulierungen mit Alkylbetainen oder Alkylsultainen bessere Eigenschaften aufweisen als entsprechende Formulierungen mit Kokosamidopropylbetain. Dies wird explizit auch experimentell belegt: Der Tabelle 1 (Seite 28) ist in den Spalten mit den Vergleichsversuchen ("Control 1" und "Control 2") zu entnehmen, dass der Versuch, Kokosamidopropylbetain zur Verdickung von Aniontensiden, u.a. Disalz, einzusetzen, scheitert: es wurden extrem schlechte Viskositätswerte erzielt (<100 mPas bzw. 200 mPas). Dementsprechend würde der Fachmann, der auf der Suche nach Viskositäts-steigernden Substanzen für wäßrige Formulierungen mit einem Gehalt an anionischen Tensiden, u.a. Disalzen, ist, auf Grund der Lehre dieses Dokumentes Amidoalkylbetainen nicht in Betracht ziehen.

### Beschreibung der Erfindung

Die komplexe Aufgabe der vorliegenden Erfindung hat darin bestanden, wäßrige Tensid-Zusammensetzungen bereitzustellen, die sich durch die im Folgenden genannten Eigenschaften auszeichnen, wobei jede dieser Eigenschaften ein technisches Merkmal darstellt:
- Gute Transparenz, worunter im Rahmen der vorliegenden Erfindung zu verstehen ist, dass die wäßrigen Tensid-Zusammensetzungen bei der quantitativen Bestimmung mittels eines TurbiScan MA 2000 (Messgerät der Firma Formulaction) bei 23°C eine mittlere Transmission von wenigstens 80%, vorzugsweise von wenigstens 85% und insbesondere von wenigstens 88% aufweisen.
- Ausreichend hohe Viskosität, worunter im Rahmen der vorliegenden Erfindung ein Wert von 1000 mPas oder höher verstanden wird (gemessen mit einem Brookfield RV Laborrheometer bei 23°C, 12 U/min, Spindelset RV 02 bis 07 (Spindelauswahl je nach Viskositätbereich)). "mPas" bedeutet bekanntlich Millipascalsekunden.
- Lagerstabilität bei Raumtemperatur (23 °C) über mindestens 8 Wochen, ohne dass irgendwelche sichtbaren Veränderungen (etwa Austrübungen, Verfärbungen, Phasentrennungen, Verlust der Transparenz und dergleichen) auftreten.
- Gutes Schaumvermögen. Hierzu sei angemerkt, dass im Bereich der Kosmetik unter Schaumvermögen verschiedene Aspekte verstanden werden können, wobei insbesondere Schaumvolumen, Schaumstabilität, Schaumelastizität, Wassergehalt des Schaumes, optische Merkmale des Schaumes wie beispielsweise die Porengröße, sowie die Schaum-Sensorik zur Beurteilung des Schaumes herangezogen werden können. Besonders wünschenswert ist, dass eine tensidische Formulierung ein großes Schaumvolumen während des Anschäumens aufweist. In der Praxis findet das Anschäumen in einem relativ kurzen Zeitraum statt (von wenigen Sekunden bis zu einer Minute). Typischerweise wird beim Anschäumen ein Duschgel oder ein Shampoo durch Reiben zwischen Händen, Haut und/oder Haaren verteilt und zum Schäumen gebracht. Ein exzellentes Anschäumverhalten ist im Rahmen der vorliegenden Erfindung für ein gutes Schaumvermögen von grundlegender Bedeutung. Im Labor kann das Anschäumverhalten einer wässrigen Tensidlösung z.B. dadurch beurteilt werden, dass man in einer vergleichbar kurzen Zeitspanne die Lösung durch Rühren, Schütteln, Pumpen, Durchperlen eines Gasstroms oder auf andere Weise in Bewegung versetzt. Der im Rahmen der vorliegenden Erfindung verwendete Schaumtest ist im Beispielteil näher beschrieben.
- Hydrolysestabilität im sauren pH-Bereich, insbesondere bei pH-Werten von 5,8 oder weniger).
Gegenstand der Erfindung sind zunächst wäßrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Amidoalkylbetaine (B)** der allgemeinen Formel (II),

   R²-CO-NH-(CH₂)_{y}-N⁺(CH₃)₂-CH₂-COO (II)

   worin der Rest R² einen linearen oder verzweigten Alkyl- oder Alkenylrest mit 7 bis 19 C-Atomen bedeutet und der Index y eine ganze Zahl im Bereich 2 bis 4 ist,
- **Wasser,**
wobei folgende Maßgaben gelten:
- In Bezug auf die Verbindungen (A) gilt, dass der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger liegt;
- in Bezug auf die Verbindungen (B) gilt, dass der Anteil der Verbindungen (B), bei denen der Rest R² ein Alkenylrest ist, - bezogen auf die Gesamtmenge der Verbindungen (B) in den wäßrigen Tensid-Zusammensetzungen - bei 3 Gew.-% oder weniger liegt;
- der Gehalt der wäßrigen Tensid-Zusammensetzungen an den Verbindungen (A) und (B) liegt - bezogen auf die gesamte wäßrige Tensid-Zusammensetzung - bei mindestens 5 Gew.-%;
- sofern die wäßrigen Tensid-Zusammensetzungen ein oder mehrere Estersulfonate (E) der allgemeinen Formel (V),

   R⁵CH(SO₃M⁵)COOR⁶ (V)

   worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁶ logischerweise erst ab 3 C-Atomen ein Alkenylrest sein oder verzweigt sein kann, und der Rest M⁵ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen;
- das Gewichtsverhältnis der Verbindungen (A) : (B) in den wäßrigen Tensid-Zusammensetzungen liegt im Bereich von 1 : 1,5 bis 1 : 5;
- der pH-Wert der wäßrigen Tensid-Zusammensetzungen liegt bei 5,8 oder weniger;
- die Viskosität der wäßrigen Tensid-Zusammensetzungen - gemessen mit einem Brookfield RV Laborrheometer bei 23°C , 12 U/min, Spindelset RV 02 bis 07 (Spindelauswahl je nach Viskositätbereich) - liegt bei 1000 mPas oder höher;
- die mittlere Transmission der wäßrigen Tensid-Zusammensetzungen bei 23°C - gemessen mit einem TurbiScan MA 2000 - liegt bei wenigstens 80%.

Der Klarheit halber sei festgestellt, dass die wäßrige Tensid-Zusammensetzung, die zur Bestimmung der mittleren Transmission herangezogen wird, sämtliche oben genannten Parameter erfüllen muss.

Überraschenderweise wurde die obengenannte komplexe Aufgabe durch die erfindungsgemäßen Tensid-Zusammensetzungen in ausgezeichneter Weise gelöst. Dabei wurden aus dem Stand der Technik bekannte Vorurteile überwunden. Es war ferner nicht vorhersehbar und mithin höchst überraschend, dass sich Disalze bei Kombination mit Amidoalkylbetainen in erheblich höheren Konzentrationen einsetzen lassen, erkennbar daran, dass die wäßrigen Zusammensetzungen transparent sind und nicht trübe. Überraschend ist ebenfalls - gerade auch angesichts des oben zitierten Standes der Technik, der den Einsatz von Disalz zur Viskositäts-Senkung lehrt, sowie des ebenfalls oben zitierten Standes der Technik, der vom Einsatz von Kokosamidopropylbetain ausdrücklich abrät - , das Auftreten hoher Viskositäten beim Einsatz einer Kombination von Disalzen und Amidoalkylbetainen.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wäßrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Ferner gilt - wie ebenfalls oben angegeben - die Maßgabe, dass der Anteil der Verbindungen (A) in den wäßrigen Tensid-Zusammensetzungen, bei denen der Rest R¹ ein Alkenylrest ist, - bezogen auf die Gesamtmenge der Verbindungen (A) - bei 3 Gew.-% oder weniger liegt.

In einer bevorzugten Ausführungsform bedeutet der Rest R¹ in der Formel (I) einen gesättigten, linearen Rest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 90 Gew.-% oder mehr liegt. Vorzugsweise bedeuten die Reste M¹ und M² in der Formel (I) Na.
Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wäßriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten im Kapitel "Bevorzugte Ausführungsformen" beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.%,
- der Gehalt an (C) liegt im Bereich von 0 bis 20 Gew.%,
- der Gehalt an (D) liegt im Bereich von 0 bis 20 Gew.%,
mit der Maßgabe, dass die Summe der Komponenten (A), (C) und (D) in dieser Mischung 100 Gew.% beträgt.

Ganz besonders sind solche technischen Mischungen bevorzugt, bei wie folgt zusammengesetzt sind:
- Der Gehalt an (A) liegt im Bereich von 70 bis 80 Gew.%,
- der Gehalt an (C) liegt im Bereich von 10 bis 15 Gew.%,
- der Gehalt an (D) liegt im Bereich von 10 bis 15 Gew.%,
mit der Maßgabe, dass die Summe der Komponenten (A), (C) und (D) in dieser Mischung 100 Gew.% beträgt.

### Zu den Verbindungen (B)

Die Verbindungen (B), die im Rahmen der vorliegenden Erfindung als **Amidoalkylbetaine** bezeichnet werden, sind für die erfindungsgemäßen wäßrigen Tensid-Zusammensetzungen obligatorisch. Sie haben die oben angegebene Formel (II)

R²-CO-NH-(CH₂)_{y}-N⁺(CH₃)₂-CH₂-COO⁻ (II)

worin der Rest R² einen linearen oder verzweigten Alkyl- oder Alkenylrest mit 7 bis 19 C-Atomen bedeutet und der Index y eine ganze Zahl im Bereich 2 bis 4 ist. Ferner gilt - wie ebenfalls oben angegeben - die Maßgabe, dass der Anteil der Verbindungen (B), bei denen der Rest R² ein Alkenylrest ist, - bezogen auf die Gesamtmenge der Verbindungen (B) in den wäßrigen Tensid-Zusammensetzungen - bei 3 Gew.-% oder weniger liegt.

Die Verbindungen (B) können nach allen dem Fachmann einschlägig bekannten Methoden hergestellt werden.

In einer Ausführungsform bedeutet der Index y in der Formel (II) die Zahl 3.

In einer Ausführungsform bedeutet Rest R² in der Formel (II) einen gesättigten, linearen Rest mit 11 bis 17 C-Atomen, wobei in Bezug auf die Verbindungen (B) gilt, dass der Anteil der Verbindungen (B), bei denen der Rest R² ein Undecyl- oder ein Tridecylrest ist, - bezogen auf die Gesamtmenge der Verbindungen (B) - bei 60 Gew.-% oder mehr liegt.

In einer bevorzugten Zusammensetzung handelt es sich in Bezug auf die Verbindungen (B) um Kokosamidopropylbetain. Es handelt sich um ein technisch verfügbares Produkt, das typischerweise in zwei Schritten hergestellt wird:
Zunächst setzt man Kokosfettsäure mit Dimethylaminopropylamin (DMAPA, chemische Formel NH₂-(CH₂)₃-N(CH₃)₂) um. Das dabei resultierende Amid wird dann in einem zweiten Schritt mit Natriumchloracetat (chemische Formel Cl-CH₂-COONa) in Gegenwart von NaOH umgesetzt, wobei unter Abspaltung von NaCl eine Quarternierung erfolgt. Das so erhältliche Produkt technischer Qualität kann neben Kokosamidopropylbetain und NaCl herstellbedingt als Nebenprodukte Glycerin, Partialglyceride, Glykolsäure, Diglykolsäure und freie Fettsäure enthalten, wobei sich der Gehalt dieser Nebenprodukte durch die Wahl geeigneter Herstellbedingungen vermindern lässt. Gewünschten falls können diese Nebenprodukte auch durch zusätzliche Reinigungsschritte Aufreinigung in ihrem Gehalt weiter reduziert oder ganz eliminiert werden.

### Bevorzugte Ausführungsformen

In einer Ausführungsform enthalten die erfindungsgemäßen wäßrigen Tensid-Zusammensetzungen neben den Verbindungen (A), (B) und Wasser zusätzlich ein oder mehrere **Verbindungen (C)** der allgemeinen Formel (III)

R⁴COOM³ (III)

In der Formel (III) bedeutet der Rest R⁴ einen linearen oder verzweigten Alkyl- oder Alkenylrest mit 7 bis 19 C-Atomen und der Reste M³ wird ausgewählt aus der Gruppe H, Li, Na, K, Ca/2, Mg/2, Ammonium und Alkanolamine.

In einer Ausführungsform enthalten die erfindungsgemäßen wäßrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁴)₂SO₄ (IV)

wobei M⁴ ausgewählt wird aus der Gruppe Li, Na, K, Ca/2, Mg/2, Ammonium und Alkanolamin.

Bei den Resten M¹ und M² der Verbindungen (A), dem Rest M³ der Verbindungen (C) und dem Rest M⁴ der Verbindungen (D) kann es sich um Alkanolamine handeln. Besonders bevorzugt sind dabei Monoethanolamin, Diethanolamin, Triethanolamin und Mono-Isopropanolamin.

In einer bevorzugten Ausführungsform enthalten die erfindungsgemäßen wäßrigen Tensid-Zusammensetzungen die Verbindungen (A), (B), (C) und (D). Dabei ist es besonders bevorzugt, wenn M¹ und M² der Verbindungen (A), der Rest M³ der Verbindungen (C) und der Rest M⁴ der Verbindungen (D) die Bedeutung Na (Natrium) hat.

Wie oben ausgeführt liegt der Gehalt der Zusammensetzungen an den Verbindungen (A) und (B) - bezogen auf die gesamte Zusammensetzung - bei mindestens 5 Gew.-%. Vorzugsweise liegt der Gehalt der Zusammensetzungen an den Verbindungen (A) und (B) - bezogen auf die gesamte Zusammensetzung - im Bereich von 5 bis 50 Gew.-% , insbesondere im Bereich von 5 bis 20 Gew.-% und besonders bevorzugt im Bereich von 8 bis 12 Gew.-%.

In einer Ausführungsform liegt das Gewichtsverhältnis der Verbindungen (A) : (B) in den Zusammensetzungen im Bereich von 1 : 3 bis 1 : 4.

In einer Ausführungsform liegt der pH-Wert der Zusammensetzungen im Bereich von 4,3 bis 4,7.

Die Viskosität der wäßrigen Tensid-Zusammensetzungen - gemessen mit einem Brookfield RV Laborrheometer bei 23°C, 12 U/min, Spindelset RV 02 bis 07 (Spindelauswahl je nach Viskositätsbereich) - liegt vorzugsweise bei 2000 mPas oder höher.

Gewünschtenfalls können die erfindungsgemäßen wäßrigen Tensid-Zusammensetzungen zusätzlich ein oder mehrere weitere Tenside enthalten, die strukturell nicht zu den oben genannten Verbindungehn (A), (B), (D) oder (E) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen. Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

### Beispiele

### Eingesetzte Substanzen

### VE-Wasser = vollentsalztes Wasser

**SFA-I:** alpha-Sulfofettsäuredisalz technischer Qualität auf Basis von nativer C_{12/14}-Fettsäure, Zusammensetzung: 74 Gew.% Dinatrium-2-Sulfolaurat, 13 Gew.% Natrium-Laurat, 11 Gew.% Natriumsulfat, 2 Gew.-% Wasser. Die Bezeichnung "Laurat" bedeutet hierbei, dass das C_{12/14}-Gewichtsverhältnis der Mischung der zu Grunde liegenden nativen Fettsäuren 70 : 30 beträgt.

**SFA-II:** aufgereinigtes alpha-Sulfofettsäuredisalz auf Basis von nativer C_{12/14}-Fettsäure, Zusammensetzung: 90 Gew.% Dinatrium-2-Sulfolaurat, 5 Gew.% Natrium-Laurat, 0,2 Gew.% Natriumsulfat, 4,8 Gew.-% Wasser. Die Bezeichnung "Laurat" bedeutet hierbei, dass das C_{12/14}-Gewichtsverhältnis der Mischung der zu Grunde liegenden nativen Fettsäuren 70 : 30 beträgt.

**Dehyton PK 45:** Cocamidopropylbetaine, 37% Aktivsubstanz (Fa. BASF PCN)

### Mess- und Prüfmethoden

**pH-Wert:** Unter Einsatz eines handelsüblichen pH-Meters, wurde der pH-Wert direkt in der Formulierung, also der wäßrigen Tensidzusammensetzung, gemessen.

**Viskosität:** Die Viskositäten der wäßrigen Tensid-Zusammensetzungen wurde mit einem Brookfield RV Laborrheometer bei 23°C, 12 U/min, Spindelset RV 02 bis 07 (Spindelauswahl nach Viskositätsbereich) gemessen.

Hinweis: Bei Viskositätsangaben, die mit (*) gekennzeichnet sind, handelt es sich um beinahe schnittfeste Gele, die eigentlich mit dem Brookfield-Rheometer nicht mehr vermessen werden können, da die Spindeln durchrutschen und das Gel durchschneiden. Die hier mit dem Zusatz (*) angegebenen Werte wurden daher bei sehr geringer Drehzahl (0,6 U/min) ermittelt, es handelt sich zwar lediglich um grobe Anhaltswerte, die gleichwohl einen Aussagewert haben.

**Homogenität und Aussehen:** Die Beurteilung der Homogenität und des Aussehens der wäßrigen Tensid-Zusammensetzungen erfolgte visuell (mit bloßem Auge) in 125ml Weithals-Glasflaschen. Beurteilt wurde dabei zunächst die Homogenität. Unter Homogenität wird im Rahmen der vorliegenden Erfindung verstanden, dass keine mit bloßem Auge sichtbaren Aufrahmung oder ein Bodensatz auftritt. Sofern die Zusammensetzungen als homogen beurteilt wurden, wurde auch ihr Aussehen beurteilt und beispielsweise mit Attributen wie leicht opak (jedoch immer noch deutlich durchscheinend) bis wasserklar charakterisiert.

**Transparenz:** Die quantitative Bestimmung der Transparenz einiger wäßriger Tensid-Zusammensetzungen erfolgte mit einem TurbiScan MA 2000 (Firma Formulaction). Dabei wurden zunächst 5ml-Proben der zu prüfenden wäßrigen Tensid-Zusammensetzungen in die gerätespezifische Messzelle gegeben und für 24 Stunden bei Raumtemperatur (23°C) stehen gelassen bis alle Luftblasen ausgetreten waren. Danach wurde die Transmission des eingestrahlten Lichts (Wellenlänge 850 nm) über eine Probenhöhe von 20 mm bis 50 mm gemessen. Die Auswertung erfolgte mit der vom Hersteller des Messgeräts mitgelieferten Software Turbisoft (Version 1.2.1.): Für jede Messung wird von der Software ein Mittelwert der Transmission (in %) über die Probenhöhe ausgegeben. Dieser Mittelwert wird im Rahmen der vorliegenden Anmeldung mittlere Transmission genannt. Dabei wurde die Transmissionsmessung bei jeder Probe 3-mal wiederholt und aus den dabei erhaltenen Werten für die mittlere Transmission der zahlenmäßige Mittelwert gebildet. Dieser Wert ist in der Tabelle 1 in der Spalte "Transparenz" angegebenen (Zahlenmittel der Versuchsdaten der mittleren Transmission aus drei Messungen).

Wo in den Tabellen kein Transmissionswert angegeben ist, erfolgte die Beurteilung ausschließlich mit bloßem Auge in einer 125ml Weithalsglasflasche.

**Schaumtest:** Die wäßrigen Tensid-Zusammensetzungen wurden mit Leitungswasser (ca. 13,8°dH, mit HCl eingestellt auf pH 4,5-4,6) im Gewichtsverhältnis 1:9 verdünnt und auf 30°C temperiert. 100g der so hergestellten wäßrigen Lösungen wurden mit einer Meiserscheibe in einem zylindrischen 800-ml-Becherglas niedriger Form (Durchmesser 10,5 cm) bei 2000 Umdrehungen/min 10s lang aufgeschäumt. Die Schaumhöhe in Zentimetern (cm) wurde abgelesen. Es wurde eine 3-fach Bestimmung durchgeführt. Als Ergebnis wurde der Mittelwert mit Standardabweichung angegeben.

### Lagerstabilität:

Die Tensid-Zusammensetzungen wurden über einen Zeitraum von 8 Wochen bei 23°C gelagert. Danach erfolgte die Prüfung der beiden Parameter Homogenität und Aussehen der Zusammensetzungen. Die Zusammensetzungen wurden dann als lagerstabil angesehen, wenn beide Parameter über den gesamten Zeitraum von 8 Wochen unverändert blieben.

### Beispiele

### Beispiel 1: Hochviskose, transparente Gel-Formulierung (Herstellbeispiel)

Herstellung (Ansatzgröße 200 g): 8,1 g SFA-I und 48,6 g Dehyton PK 45 wurden unter Rühren bei 23 °C in 143,2 g VE-Wasser verlöst (vergl. Tabelle 1). Danach wurde durch Zugabe von Citronensäure (50%ige Lösung) der pH-Wert auf 4,7 eingestellt.

Die beurteilten Parameter (Viskosität, Homogenität, Aussehen, Schaum, Lagerstabilität, Transparenz) können der Tabelle 1 entnommen werden.

Beispiel 1 zeigt, dass mit diesem Tensidsystem ohne Zusatz von Verdickungsmitteln sehr hohe Viskositäten erreicht werden können.

Im Schaumtest zeigte diese Formulierung gemäß Beispiel 1 eine Schaumhöhe von 7,6 cm +/-0,23 cm und schäumte damit signifikant stärker als die jeweiligen Einzeltenside (vergleiche Vergleichsbeispiele 1 und 2).

Über die Beurteilung der für die Lagerstabilität maßgeblichen beiden oben genannten Parameter Homogenität und Aussehen hinaus wurde zusätzlich folgender Test durchgeführt: Die Tensid-Zusammensetzung wurden im Gefrierschrank bei einer Temperatur von -8 °C bis zur vollständigen Verfestigung abgekühlt. Danach ließ man die Proben bei einer Temperatur von 23 °C wieder auftauen. Anschließend wurden die Homogenität und das Aussehen der aufgetauten Probe beurteilt; beide Parameter waren unverändert, d.h. die Proben waren homogen und klar.

### Beispiel 2: Transparente Formulierung mit Duschgel-Viskosität (Anwendungsbeispiel)

Herstellung wie Beispiel 1, jedoch mit geänderten Mengen der eingesetzten Komponenten (siehe Tabelle 1). pH-Wert Einstellung wie in Beispiel 1 mit Citronensäure (auf den in Tabelle 1 angegebenen Wert). Die beurteilten Parameter (Viskosität, Homogenität, Aussehen, Lagerstabilität, Transparenz) können der Tabelle 1 entnommen werden.

Die Viskosität der Rezeptur gemäß Beispiel 2 war deutlich niedriger als in Beispiel 1. Der Wert von 4100 mPas wäre beispielsweise für ein Duschgel geeignet.

### Beispiel 3: Hochviskose, transparente Gel-Formulierung mit niedrigerem Aktivsubstanzgehalt (Anwendungsbeispiel)

Herstellung wie Beispiel 1, jedoch mit geänderten Mengen der eingesetzten Komponenten (siehe Tabelle 1). pH-Wert Einstellung wie in Beispiel 1 mit Citronensäure (auf den in Tabelle 1 angegebenen Wert). Die beurteilten (Viskosität, Homogenität, Aussehen, Lagerstabilität, Transparenz) können der Tabelle 1 entnommen werden.

Beispiel 3 zeigt, dass der Verdickungseffekt auch bei reduziertem Aktivsubstanzgehalt (hier nur 8 Gew.%) noch vorhanden ist.

**Tabelle 1:**

| | Beispiel 1 | Beispiel 2 | Beispiel 3 |
|---|---|---|---|
| SFA-I | 8,1 g | 5,4 g | 5,4 g |
| Dehyton PK 45 | 48,6 g | 54,1 g | 32,4 g |
| VE-Wasser | 143,3 g | 140,5 g | 162,2 g |
| pH Wert | 4,7 | 4,5 | 4,7 |
| Viskosität | Gel > 230000 mPas(*) | 4100 mPas | Gel >180000 mPas(*) |
| Homogenität | homogen | homogen | homogen |
| Aussehen | klar | klar | leicht opak |
| Schaumtest | 7,6 cm | | |
| Lagerstabilität | lagerstabil | lagerstabil | lagerstabil |
| Transparenz | 92,9% | 92,3% | 89,7% |
| Aktivsubstanzgehalt | 12% | 12% | 8% |
| Verhältnis (A) : (B) (Gew.% Aktivsubstanz) | 1:3 | 1:5 | 1:3 |

Hinweis: Unter Aktivsubstanz wird in Tabelle 1 sowie in den Tabellen 2 und 3 die Summe der anionischen Tenside und der amphoteren Tenside verstanden. Die Angaben sind in Gew.-%.

### Beispiel 4: Viskose, transparente Gel-Formulierung

Herstellung wie Beispiel 1, jedoch mit geänderten Mengen der eingesetzten Komponenten sowie Verwendung von SFA-II anstelle von SFA-I (siehe Tabelle 2). pH-Wert Einstellung wie in Beispiel 1 mit Citronensäure (auf den in Tabelle 2 angegebenen Wert von 4,7). Die beurteilten Parameter (Viskosität, Homogenität, Aussehen, Lagerstabilität) können der Tabelle 2 entnommen werden.

**Tabelle 2:**

| | Beispiel 4 |
|---|---|
| SFA-II | 6,7 g |
| Dehyton PK 45 | 48,6 g |
| Dehyton AB 30 | |
| Texapon NSO | |
| VE-Wasser | 144,7 g |
| pH Wert | 4,7 |
| Viskosität | Gel >30000 mPas(*) |
| Homogenität | homogen |
| Aussehen | klar |
| Lagerstabilität | lagerstabil |
| Aktivsubstanzgehalt | 12% |
| Verhältnis (A) : (B) (Gew.% Aktivsubstanz) | 1:3 |

### Vergleichsbeispiel 1: Ausschließlich SFA-I enthaltende Formulierung

Herstellung wie Beispiel 1, jedoch mit geänderten Mengen der eingesetzten Komponenten (siehe Tabelle 3). pH-Wert Einstellung wie in Beispiel 1 mit Citronensäure (auf den in Tabelle 3 angegebenen Wert). Die beurteilten Parameter (Viskosität, Homogenität, Aussehen, Schaum) können der Tabelle 3 entnommen werden.

Das vorliegende Vergleichsbeispiel zeigt, dass eine Formulierung, die ausschließlich SFA-I enthält, wesentliche Ziele der Aufgabenstellung nicht erreicht. Weder ließ sich das anionische Tensid FSA-I in Wasser verlösen, noch zeigte sich eine verdickende Wirkung. Im Schaumtest erreicht diese Formulierung lediglich eine Schaumhöhe von 5,1 cm +/- 0,36 cm, was signifikant schlechter ist, als die in Beispiel 1 gemessene Schaumhöhe.

### Vergleichsbeispiel 2: Ausschließlich Dehyton PK 45 enthaltende Formulierung

Herstellung wie Beispiel 1, jedoch mit geänderten Mengen der eingesetzten Komponenten (siehe Tabelle 3). pH-Wert Einstellung wie in Beispiel 1 mit Citronensäure (auf den in Tabelle 3 angegebenen Wert). Die beurteilten Parameter (Viskosität, Homogenität, Aussehen, Schaum) können der Tabelle 3 entnommen werden.

Das vorliegende Vergleichsbeispiel zeigt, dass eine Formulierung, die ausschließlich Dehyton PK 45 enthält, wesentliche Ziele der Aufgabenstellung nicht erreicht. Weder wurde eine verdickende Wirkung erreicht, noch erreichte diese Formulierung einen akzeptablen Wert im Schaumtest. Die gemessene Schaumhöhe betrug 5,5 cm +/- 0 cm, was signifikant schlechter ist, als die in Beispiel 1 gemessene Schaumhöhe.

**Tabelle 3:**

| | Vergleichsbeispiel 1 | Vergleichsbeispiel 2 | Vergleichsbeispiel 3 |
|---|---|---|---|
| SFA-I | 32,4 g | | 8,1 g |
| Dehyton PK 45 | | 64,9 g | 48,6 g |
| Deyhton AB 30 | | | |
| Texapon NSO | | | |
| VE-Wasser | 167,6 g | 135,1 g | 143,3 g |
| pH Wert | 4,5 | 4,5 | 6,0 |
| Viskosität | unmessbar gering, überstehende Phase wasserdünn | 100 mPas | 100 mPas |
| Homogenität | nicht homogen | homogen | nicht homogen |
| Aussehen | milchig trübe Dispersion; trennte sich nach einiger Zeit in klare überstehende Phase und weißes Sediment | klar | trübe Dispersion; trennte sich nach einiger Zeit und bildete Bodensatz |
| Aktivsubstanzgehalt | 12% | 12% | 12% |
| Schaumtest | 5,1 cm | 5,5 cm | |
| Verhältnis (A) : (B) (Gew.% Aktivsubstanz) | 1:0 | 0:1 | 1:3 |

### Vergleichsbeispiel 3: Formulierung mit nicht erfindungsgemäßem pH-Wert

Herstellung wie Beispiel 1, jedoch mit geänderten Mengen der eingesetzten Komponenten (siehe Tabelle 3). pH-Wert Einstellung wie in Beispiel 1 mit Citronensäure (auf den in Tabelle 3 angegebenen Wert von 6,0). Die beurteilten Parameter (Viskosität, Homogenität, Aussehen) können der Tabelle 3 entnommen werden.

Das vorliegende Vergleichsbeispiel zeigt, dass eine Formulierung, deren pH-Wert nicht erfindungsgemäß ist, wesentliche Ziele der Aufgabenstellung nicht erreicht. Weder ergab sich eine klare Lösung, noch wurde eine verdickende Wirkung erreicht. Dies demonstriert, dass der pH-Wert ein kritischer, d.h. erfindungswesentlicher Parameter dafür ist.

## Patentansprüche

1. Wäßrige Tensid-Zusammensetzungen enthaltend
• ein oder mehrere **alpha-Sulfofettsäuredisalze (A)** der allgemeinen Formel (I),
R¹CH(SO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkano lamin,
• ein oder mehrere **Amidoalkylbetaine (B)** der allgemeinen Formel (II),
R²-CO-NH-(CH₂)_{y}-N⁺(CH₃)₂-CH₂-COO⁻ (II)
worin der Rest R² einen linearen oder verzweigten Alkyl- oder Alkenylrest mit 7 bis 19 C-Atomen bedeutet und der Index y eine ganze Zahl im Bereich 2 bis 4 ist,
• **Wasser,**
wobei folgende Maßgaben gelten:
• In Bezug auf die Verbindungen (A) gilt, dass der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger liegt;
• in Bezug auf die Verbindungen (B) gilt, dass der Anteil der Verbindungen (B), bei denen der Rest R² ein Alkenylrest ist, - bezogen auf die Gesamtmenge der Verbindungen (B) in den wäßrigen Tensid-Zusammensetzungen - bei 3 Gew.-% oder weniger liegt;
• der Gehalt der wäßrigen Tensid-Zusammensetzungen an den Verbindungen (A) und (B) liegt - bezogen auf die gesamte wäßrigen Tensid-Zusammensetzung - bei mindestens 5 Gew.-%;
• sofern die wäßrigen Tensid-Zusammensetzungen ein oder mehrere **Estersulfonate (E)** der allgemeinen Formel (V),
R⁵CH(SO₃M⁵)COOR⁶ (V)
worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁶ logischerweise erst ab 3 C-Atomen ein Alkenylrest sein oder verzweigt sein kann, und der Rest M⁵ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr vorliegen müssen;
• das Gewichtsverhältnis der Verbindungen (A) : (B) in den wäßrigen Tensid-Zusammensetzungen liegt im Bereich von 1 : 1,5 bis 1 : 5;
• der pH-Wert der wäßrigen Tensid-Zusammensetzungen liegt bei 5,8 oder weniger;
• die Viskosität der wäßrigen Tensid-Zusammensetzungen - gemessen mit einem Brookfield RV Laborrheometer bei 23°C, 12 U/min, Spindelset RV 02 bis 07 (Spindelauswahl je nach Viskositätbereich) - liegt bei 1000 mPas oder höher;
• die mittlere Transmission der wäßrigen Tensid-Zusammensetzungen bei 23°C - gemessen mit einem TurbiScan MA 2000 - liegt bei wenigstens 80%.

2. Zusammensetzungen nach Anspruch 1, wobei der Index y in der Formel (II) die Zahl 3 bedeutet.

3. Zusammensetzungen nach Anspruch 1 oder 2, wobei der Rest R² in der Formel (II) einen gesättigten, linearen Rest mit 11 bis 17 C-Atomen bedeutet, wobei in Bezug auf die Verbindungen (B) gilt, dass der Anteil der Verbindungen (B), bei denen der Rest R² ein Undecyl- oder ein Tridecylrest ist, - bezogen auf die Gesamtmenge der Verbindungen (B) - bei 60 Gew.-% oder mehr liegt.

4. Zusammensetzungen nach einem der Ansprüche 1 bis 3, wobei es sich in Bezug auf die Verbindungen (B) um Kokosamidopropylbetain handelt.

5. Zusammensetzungen nach einem der Ansprüche 1 bis 4, wobei der Rest R¹ in der Formel (I) einen gesättigten, linearen Rest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

6. Zusammensetzungen nach einem der Ansprüche 1 bis 5, wobei die Reste M¹ und M² Na bedeuten.

7. Zusammensetzungen nach einem der Ansprüche 1 bis 6, wobei die Zusammensetzungen zusätzlich ein oder mehrere **Verbindungen (C)** der allgemeinen Formel (III)
R⁴COOM³ (III)
enthält, worin der Rest R⁴ einen linearen oder verzweigten Alkyl- oder Alkenylrest mit 7 bis 19 C-Atomen bedeutet und der Reste M³ ausgewählt wird aus der Gruppe H, Li, Na, K, Ca/2, Mg/2, Ammonium und Alkanolamin.

8. Zusammensetzungen nach einem der Ansprüche 1 bis 7, wobei die Zusammensetzungen zusätzlich ein oder mehrere **anorganische Salze der Schwefelsäure (D)** der allgemeinen Formel (IV)
(M⁴)₂SO₄ (IV)
Enthält, wobei M⁴ ausgewählt wird aus der Gruppe Li, Na, K, Ca/2, Mg/2, Ammonium und Alkanolamin.

9. Zusammensetzungen nach einem der Ansprüche 1 bis 8, wobei der Gehalt der Zusammensetzungen an den Verbindungen (A) und (B) - bezogen auf die gesamte Zusammensetzung - im Bereich von 8 bis 12 Gew.-% liegt.

10. Zusammensetzungen nach einem der Ansprüche 1 bis 9, wobei das Gewichtsverhältnis der Verbindungen (A) : (B) in den Zusammensetzungen im Bereich von 1 : 3 bis 1 : 4 liegt.

11. Zusammensetzungen nach einem der Ansprüche 1 bis 10, wobei der pH-Wert der Zusammensetzungen im Bereich von 4,3 bis 4,7 liegt.

12. Zusammensetzungen nach einem der Ansprüche 1 bis 11, wobei die Viskosität der Zusammensetzungen - gemessen mit einem Brookfield RV Laborrheometer bei 23°C , 12 U/min, Spindelset RV 02 bis 07 (Spindelauswahl je nach Viskositätbereich) - 2000 mPas oder höher ist.

13. Verwendung der Zusammensetzungen nach einem der Ansprüche 1 bis 12 für kosmetische Mittel sowie Wasch- und Reinigungsmittel.

14. Verwendung der Zusammensetzungen nach einem der Ansprüche 1 bis 12 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

15. Verwendung der Zusammensetzungen nach einem der Ansprüche 1 bis 12 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.

## Claims

1. An aqueous surfactant composition comprising
• one or more **alpha-sulfo fatty acid disalts (A)** of the general formula (I),
R¹CH(SO₃M¹)COOM² (I)
in which the radical R¹ is a linear or branched alkyl or alkenyl radical with 6 to 18 carbon atoms and the radicals M¹ and M² - independently of one another - are selected from the group H, Li, Na, K, Ca/2, Mg/2, ammonium and alkanolamine,
• one or more **amidoalkylbetaines (B)** of the general formula (II),
R²⁻CO-NH- (CH₂)_{y}-N⁺(CH₃)₂-CH₂-COO- (II)
in which the radical R² is a linear or branched alkyl or alkenyl radical with 7 to 19 carbon atoms and the index y is an integer in the range 2 to 4,
• **water,**
where the following provisos apply:
• with regard to compounds (A) it is the case that the fraction of the compounds (A) in which the radical R¹ is an alkenyl radical - based on the total amount of the compounds (A) in the aqueous surfactant composition - is 3% by weight or less;
• with regard to the compounds (B), it is the case that the fraction of the compounds (B) in which the radical R² is an alkenyl radical - based on the total amount of the compounds (B) in the aqueous surfactant composition - is 3% by weight or less;
• the content of the compounds (A) and (B) in the aqueous surfactant composition is - based on the total aqueous surfactant composition - at least 5% by weight;
• if the aqueous surfactant composition comprises one or more **ester sulfonates (E)** of the general formula (V) ,
R⁵CH(SO₃M⁵)COOR⁶ (V)
in which the radical R⁵ is a linear or branched alkyl or alkenyl radical with 6 to 18 carbon atoms and the radical R⁶ is a linear or branched alkyl or alkenyl radical with 1 to 20 carbon atoms, where the radical R⁶ can logically be an alkenyl radical or be branched only above 3 carbon atoms, and the radical M⁵ is selected from the group Li, Na, K, Ca/2, Mg/2, ammonium and alkanolamines, it is the case that the compounds (A) - based on the totality of the compounds (A) and (E) - must be present to 50% by weight or more;
• the weight ratio of the compounds (A) : (B) in the aqueous surfactant composition is in the range from 1 : 1.5 to 1 : 5;
• the pH of the aqueous surfactant composition is 5.8 or less;
• the viscosity of the aqueous surfactant composition - measured using a Brookfield RV laboratory rheometer at 23°C, 12 rpm, spindle set RV 02 to 07 (spindle choice depending on viscosity range) - is 1000 mPas or higher;
• the average transmission of the aqueous surfactant composition at 23°C - measured using a TurbiScan MA 2000 - is at least 80%.

2. The composition according to claim 1, wherein the index y in the formula (II) is the number 3.

3. The composition according to claim 1 or 2, wherein the radical R² in the formula (II) is a saturated, linear radical with 11 to 17 carbon atoms, where, with regard to the compounds (B), it is the case that the fraction of the compounds (B) in which the radical R² is an undecyl or a tridecyl radical - based on the total amount of the compounds (B) - is 60% by weight or more.

4. The composition according to any one of claims 1 to 3, where the compounds (B) are cocamidopropylbetaine.

5. The composition according to any one of claims 1 to 4, where the radical R¹ in the formula (I) is a saturated, linear radical with 10 to 16 carbon atoms, where with regard to the compounds (A) it is the case that the fraction of the compounds (A) in which the radical R¹ is a decyl or a dodecyl radical, - based on the total amount of the compounds (A) - is 90% by weight or more.

6. The composition according to any one of claims 1 to 5, where the radicals M¹ and M² are Na.

7. The composition according to any one of claims 1 to 6, wherein the composition additionally comprises one or more **compounds (C)** of the general formula (III)
R⁴COOM³ (III)
in which the radical R⁴ is a linear or branched alkyl or alkenyl radical with 7 to 19 carbon atoms and the radical M³ is selected from the group H, Li, Na, K, Ca/2, Mg/2, ammonium and alkanolamine.

8. The composition according to any one of claims 1 to 7, wherein the composition additionally comprises one or more **inorganic salts of sulfuric acid (D)** of the general formula (IV)
(M⁴)₂SO₄ (IV)
wherein M⁴ is selected from the group Li, Na, K, Ca/2, Mg/2, ammonium and alkanolamine.

9. The composition according to any one of claims 1 to 8, wherein the content of the compounds (A) and (B) in the composition - based on the total composition - is in the range from 8 to 12% by weight.

10. The composition according to any one of claims 1 to 9, wherein the weight ratio of the compounds (A) : (B) in the composition is in the range from 1 : 3 to 1 : 4.

11. The composition according to any one of claims 1 to 10, wherein the pH of the composition is in the range from 4.3 to 4.7.

12. The composition according to any one of claims 1 to 11, wherein the viscosity of the composition - measured using a Brookfield RV laboratory rheometer at 23°C, 12 rpm, spindle set RV 02 to 07 (spindle choice depending on viscosity range) - is 2000 mPas or higher.

13. The use of the composition according to any one of claims 1 to 12 for cosmetic products and also detergents and cleaners.

14. The use of the composition according to any one of claims 1 to 12 for cosmetic products in the form of hair shampoos, shower gels, soaps, syndets, washing pastes, washing lotions, scrub preparations, foam baths, oil baths, shower baths, shaving foams, shaving lotions, shaving creams and dental care products.

15. The use of the composition according to any one of claims 1 to 12 for products with a low pH for cleaning hard surfaces, such as bath and toilet cleaners and the like, and also for cleaning and/or fragrance gels for use in sanitary installations.

## Revendications

1. Compositions aqueuses de tensioactifs, contenant :
- un ou plusieurs disels d'acides alpha-sulfogras (A) de formule générale (I)
R¹CH(SO₃M¹)COOM² (I)
dans laquelle le radical R¹ signifie un radical alkyle ou alcényle linéaire ou ramifié de 6 à 18 atomes C et les radicaux M¹ et M² sont choisis, indépendamment l'un de l'autre, dans le groupe constitué par H, Li, Na, K, Ca/2, Mg/2, ammonium et alcanolamine,
- une ou plusieurs amidoalkylbétaïnes (B) de formule générale (II)
R²-CO-NH-(CH₂)_{y}-N⁺(CH₃)₂-CH₂-COO⁻ (II)
dans laquelle le radical R² signifie un radical alkyle ou alcényle linéaire ou ramifié de 7 à 19 atomes C et l'indice y est un nombre entier dans la plage allant de 2 à 4,
- de l'eau,
les conditions suivantes s'appliquant :
- en ce qui concerne les composés (A), la proportion des composés (A) dans lesquels le radical R¹ est un radical alcényle, par rapport à la quantité totale des composés (A) dans les compositions aqueuses de tensioactif, est de 3 % en poids ou moins ;
- en ce qui concerne les composés (B), la proportion des composés (B) dans lesquels le radical R² est un radical alcényle, par rapport à la quantité totale des composés (B) dans les compositions aqueuses de tensioactif, est de 3 % en poids ou moins ;
- la teneur des compositions aqueuses de tensioactif en composés (A) et (B), par rapport à l'ensemble de la composition aqueuse de tensioactif, est d'au moins 5 % en poids ;
- si les compositions aqueuses de tensioactif contiennent un ou plusieurs ester-sulfonates (E) de formule générale (V),
R⁵CH(SO₃M⁵)COOR⁶ (V)
dans laquelle le radical R⁵ signifie un radical alkyle ou alcényle linéaire ou ramifié de 6 à 18 atomes C et le radical R⁶ signifie un radical alkyle ou alcényle linéaire ou ramifié de 1 à 20 atomes C, le radical R⁶ ne pouvant logiquement être un radical alcényle ou être ramifié qu'à partir de 3 atomes C, et le radical M⁵ est choisi dans le groupe constitué par Li, Na, K, Ca/2, Mg/2, ammonium et alcanolamine, les composés (A) doivent être présents, par rapport à l'ensemble des composés (A) et (E), à hauteur de 50 % en poids ou plus ;
- le rapport en poids des composés (A):(B) dans les compositions aqueuses de tensioactif se situe dans la plage allant de 1:1,5 à 1:5 ;
- le pH des compositions aqueuses de tensioactif est de 5,8 ou moins ;
- la viscosité des compositions aqueuses de tensioactif, mesurée avec un rhéomètre de laboratoire Brookfield RV à 23 °C, 12 tours/minute, set de tiges RV 02 à 07 (la tige est choisie selon la plage de viscosité), est de 1 000 mPas ou plus ;
- la transmission moyenne des compositions aqueuses de tensioactif à 23 °C, mesurée avec un TurbiScan MA 2000, est d'au moins 80 %.

2. Compositions selon la revendication 1, dans lesquelles l'indice y dans la formule (II) signifie le nombre 3.

3. Compositions selon la revendication 1 ou 2, dans lesquelles le radical R² dans la formule (II) signifie un radical linéaire saturé de 11 à 17 atomes C, en ce qui concerne les composés (B), la proportion des composés (B) dans lesquels le radical R² est un radical undécyle ou tridécyle, par rapport à la quantité totale des composés (B), étant de 60 % en poids ou plus.

4. Compositions selon l'une quelconque des revendications 1 à 3, dans lesquelles les composés (B) sont de la cocoamidopropylbétaïne.

5. Compositions selon l'une quelconque des revendications 1 à 4, dans lesquelles le radical R¹ dans la formule (I) signifie un radical linéaire saturé de 10 à 16 atomes C, en ce qui concerne les composés (A), la proportion des composés (A) dans lesquels le radical R¹ est un radical décyle ou dodécyle, par rapport à la quantité totale des composés (A), étant de 90 % en poids ou plus.

6. Compositions selon l'une quelconque des revendications 1 à 5, dans lesquelles les radicaux M¹ et M² signifient Na.

7. Compositions selon l'une quelconque des revendications 1 à 6, dans lesquelles les compositions contiennent en outre un ou plusieurs composés (C) de formule générale (III)
R⁴COOM³ (III)
dans laquelle le radical R⁴ signifie un radical alkyle ou alcényle linéaire ou ramifié de 7 à 19 atomes C et le radical M³ est choisi dans le groupe constitué par H, Li, Na, K, Ca/2, Mg/2, ammonium et alcanolamine.

8. Compositions selon l'une quelconque des revendications 1 à 7, dans lesquelles les compositions contiennent en outre un ou plusieurs sels inorganiques de l'acide sulfurique (D) de formule générale (IV)
(M⁴)₂SO₄ (IV)
dans laquelle M⁴ est choisi dans le groupe constitué par Li, Na, K, Ca/2, Mg/2, ammonium et alcanolamine.

9. Compositions selon l'une quelconque des revendications 1 à 8, dans lesquelles la teneur des compositions en composés (A) et (B), par rapport à l'ensemble de la composition, se situe dans la plage allant de 8 à 12 % en poids.

10. Compositions selon l'une quelconque des revendications 1 à 9, dans lesquelles le rapport en poids des composés (A):(B) dans les compositions se situe dans la plage allant de 1:3 à 1:4.

11. Compositions selon l'une quelconque des revendications 1 à 10, dans lesquelles le pH des compositions se situe dans la plage allant de 4,3 à 4,7.

12. Compositions selon l'une quelconque des revendications 1 à 11, dans lesquelles la viscosité des compositions, mesurée avec un rhéomètre de laboratoire Brookfield RV à 23 °C, 12 tours/minute, set de tiges RV 02 à 07 (la tige est choisie selon la plage de viscosité), est de 2 000 mPas ou plus.

13. Utilisation des compositions selon l'une quelconque des revendications 1 à 12 pour des agents cosmétiques, ainsi que des détergents.

14. Utilisation des compositions selon l'une quelconque des revendications 1 à 12 pour des agents cosmétiques sous la forme de shampoings pour les cheveux, de gels douche, de savons, de détersifs synthétiques, de pâtes lavantes, de lotions lavantes, de préparations de gommage, de bains moussants, d'huiles pour le bain, de bains-douches, de mousses à raser, de lotions à raser, de crèmes à raser et de produits pour le soin des dents.

15. Utilisation des compositions selon l'une quelconque des revendications 1 à 12 pour des agents ayant un pH faible pour le nettoyage de surfaces dures, tels que des produits de nettoyage pour la salle de bains et les toilettes et analogues, ainsi que pour des gels nettoyants et/ou parfumés destinés à une utilisation dans des dispositifs sanitaires.
